# EUROPEAN PATENT SPECIFICATION

(11) **EP 3 359 028 B1**
(45) Date of publication and mention of the grant of the patent: **25.09.2024**
(21) Application number: 16781719.6
(22) Date of filing: 07.10.2016
(51) Int. Cl.: A61B 5/022, A61B 5/00

(54) **A DETECTION DEVICE FOR USE IN A BLOOD PRESSURE MEASUREMENT SYSTEM**
ERKENNUNGSVORRICHTUNG ZUR VERWENDUNG IN EINEM SYSTEM ZUR BLUTDRUCKMESSUNG
DISPOSITIF DE DÉTECTION DESTINÉ À ÊTRE UTILISÉ DANS UN SYSTÈME DE MESURE DE PRESSION SANGUINE

(30) Priority: 08.10.2015 EP 15188876
(43) Date of publication of application: 15.08.2018
(73) Proprietor: Koninklijke Philips N.V., 5656 AG Eindhoven (NL)
(72) Inventor: WOEHRLE, Dieter, 5656 AE Eindhoven (NL); WUNDERLE, Norbert Ernst, 5656 AE Eindhoven (NL)
(74) Representative: Philips Intellectual Property & Standards
(86) International application number: PCT/EP2016/074014
(87) International publication number: WO 2017/060436

(56) References cited:
- WO-A1-86/03114
- US-A- 4 669 485
- US-A- 5 323 782
- US-A1- 2014 257 116

## Description

### FIELD OF INVENTION

The invention is generally directed at a non-invasive blood pressure measurement system. More particularly, various inventive methods and apparatus disclosed herein relate to a detection device for verifying whether an inflatable cuff of the non-invasive blood pressure measurement system is wrapped around a measurement site of a patient and connected to the non-invasive blood pressure measurement system.

### BACKGROUND OF THE INVENTION

High blood pressure is a significant risk factor for heart attack, stroke, kidney disease, and vision loss. It often is referred to as the silent killer because it rarely causes any symptoms until considerable organ damage has occurred. For that reason, obtaining regular, accurate blood pressure readings are important to long-term health. In hospital and ambulatory setting, constant or periodic blood pressure monitoring is essential component of monitoring patient's vital signs.

For many years blood pressure has been measured using an upper arm pressure cuff and auscultation of the brachial artery to identify the appearance and disappearance of Korotkoff sounds. Although the auscultatory method employing mercury sphygmomanometers used to be regarded as the "gold standard" for blood pressure measurement, widespread implementation of the ban in use of mercury sphygmomanometers continues to diminish the role of this technique. When home monitoring was first introduced, most approached relied upon aneroid sphygmomanometers.

Increasingly, automated devices for measuring blood pressure are now used in the clinic, hospitals and by people in their homes. In addition, ambulatory blood pressure measurement devices are available that are programmed to allow blood pressure to be measured repeatedly during the day and night. The standard type of monitor for home or ambulatory use is now an oscillometric device that records pressure from the brachial artery. These have the advantage of being easy to use, because cuff placement is not as critical as with devices that use a Korotkoff sound microphone, and the oscillometric method has in practice been found to be as reliable as the Korotkoff sound method.

Nowadays, automated blood pressure monitoring has rapidly essentially replaced the traditional mercury or aneroid sphygmomanometers and the stethoscope, becoming an essential tool of patient care and home healthcare.

Conventional non-invasive blood pressure ("NIBP") measurement systems engage the sphygmo-manometric occlusive limb-cuffs that are applied around an extremity of a patient's body, e.g. wrapped around a patient's upper arm. When the NIBP system is used, the blood pressure cuff is placed around a limb of a patient and is inflated to an initial inflation pressure that fully occludes the brachial artery to temporarily prevent blood pressure flow. The cuff is then deflated from the initial inflation pressure and the pressure transducer detects pressure pulses associated with the patient's heartbeat, as blood begins to flow past the pressure cuff. Alternatively, the cuff is inflated slowly until the brachial artery is occluded completely, the pressure transducer detects pressure pulses associated with the patient's heartbeat during inflation and then the cuff is deflated rapidly.

Known NIBP systems support various cuff sizes, from neonatal to extra-large adult cuffs, where the inflation timeout has to be long enough to allow the inflation of the largest supported cuff loosely wrapped around the largest supported limb, because the NIBP system usually does not recognize which size cuff is actually connected. Consequently, the timeout makes it unsuitable for detecting whether or not the cuff is wrapped around a limb for smaller cuffs because a smaller cuff can be inflated to the target pressure faster than the timeout period even though it is not wrapped around a limb.

Not being able to detect whether the cuff is wrapped around a patient's limb ("cuff off" detection) is particularly detrimental to automatic blood pressure monitoring systems capable of programmable measurement sequences, because a clinical staff or a patient may intentionally remove or accidentally dislodge the cuff from the measurement site on the limb or disconnect the cuff from the NIBP monitor without stopping the automatic measurement mode or programmable measurement sequence. As a result, the NIBP system continues to take measurements and may obtain phantom readings due to cuff pressure oscillations caused by stretching of the cuff or movement of the cuff induced by external vibrations, etc.

US8808189B2 discloses a method of detecting the wrapping strength of a cuff that is wrapped around the limb and not detached from it. The detection is made after the pressure-volume relationship according to a change of the cuff pressure detected in the cuff wrapped around the measurement site and, equally, volume change of the cuff detected by the volume detection unit with the change of its pressure whilst it is controlled by pressurization or depressurization by the pressure control unit.

US4669485A discloses apparatus and related methods for continuous long term non-invasive measurement of the pressure of a pulsatile fluid flowing through a flexible tube, particularly human arterial blood flow, is disclosed. Specifically, the apparatus provides a continuous calibrated pressure measurement by first undertaking a "calibration" phase comprised of determining the pressure at various pre-defined conditions of flow and, in response thereto, ascertaining the values of a plurality of coefficients each of which is associated with a corresponding term in a pre-defined function that characterizes fluid pressure in relation to pulsatile displacement of the wall of the tube; and second, undertaking a "continuous monitoring" phase comprised of determining each subsequently occurring pressure value as the pre-defined function of each corresponding pulsatile wall displace- ment value, and re-initiating the calibration phase at the expiration of pre-defined time intervals which adaptively change based upon current and prior results. Furthermore, cuff integrity verification routine is performed to determine whether any air leaks occur and/or whether the cuffs are properly secured to the patient. The computer first determines the actual pumping rate, associated with each cuff at various predefined times occurring early in the pressurization. Once an actual pressurization rate is determined, a previously-determined look-up table is accessed. This table consists of minimum and maximum acceptable pressurization rates for the waveform sensor cuff and for each different permissible occlusive cuff size. For lower-than-expected pressurization rates, "loosely fitted cuff', "detached cuff, "disconnected air line" or "air line leakage" fault conditions can be identified and displayed.

US2014257116A1 relates to electronic blood pressure meters, and particularly relates to electronic blood pressure meters that measure a blood pressure using pulse waves detected from a measurement area.

US5323782A relates to an electronic blood pressure meter capable of performing an automatic pressuring operation according to the blood pressure of a subject and measuring the blood pressure rapidly with accuracy.

### SUMMARY OF THE INVENTION

The invention is defined by the independent claims. Dependent claims define advantageous embodiments.

Generally, it is an object of the present invention to enable cuff-off detection for blood pressure measurement devices. More particularly, in its various embodiments, the invention focuses on a blood pressure measurement system and apparatus capable of detecting improper functioning due to the absence of an inflatable cuff from a patient's measurement site, its improper attachment to the site, or it being disconnected from the blood pressure measurement system.

According to the first aspect of the invention, this object is addressed by a detection device as claimed in claim 1. As explained above, it is advantageous to detect whether a cuff is disconnected from a blood pressure measurement system or is unattached to the patient's limb to avoid phantom readings during a series of automatic measurements. If the inflation parameter value determined during a preceding inflation operation does not meet a predetermined criterion, the measurements are immediately stopped.

According to the invention, the corresponding inflation operation and the preceding inflation operation are sequential operations in an automatic measurement series and/or programmable measurement sequences. The "cuff off" detection is particularly important during a series of automatic measurements and programmable measurement sequences.

In various embodiments, the inflation speed-dependent parameter is defined as a period of time required during the inflation operation to bring the pressure of the cuff from the first pressure level to the second pressure level.

In some embodiments, the first pressure level is a pressure of the cuff at the start of the inflation operation. The second pressure level may be any pressure between the first pressure level and the target cuff pressure at the end of the inflation operation. Preferably, the second pressure level is substantially smaller than the target cuff pressure at the end of the inflation operation in order to stop an unnecessary inflation as soon as possible. In many embodiments, the inflation speed-dependent parameter is the first derivative (dP/dt) of the cuff pressure during inflation.

According to the second aspect, the invention provides a method for checking whether an inflatable cuff has been disconnected from or has been attached to a measurement site of a body part of a patient, as defined in the independent claim 10.

### BRIEF DESCRIPTION OF THE DRAWINGS

In the drawings, similar reference characters generally refer to the same parts throughout different views. Also, the drawings are not necessarily to scale, with the emphasis instead generally being placed upon illustrating the principles of the invention.
Fig. 1 shows a block diagram of a system for non-invasive monitoring blood pressure with the device for checking whether an inflatable cuff is attached to a patient's limb;
Fig. 2 shows a graph of the cuff inflation and deflation cycle;
Fig. 3 shows a graph of the cuff inflation process; and
Fig. 4 shows the method's flow diagram;

### DETAILED DESCRIPTION OF THE INVENTION

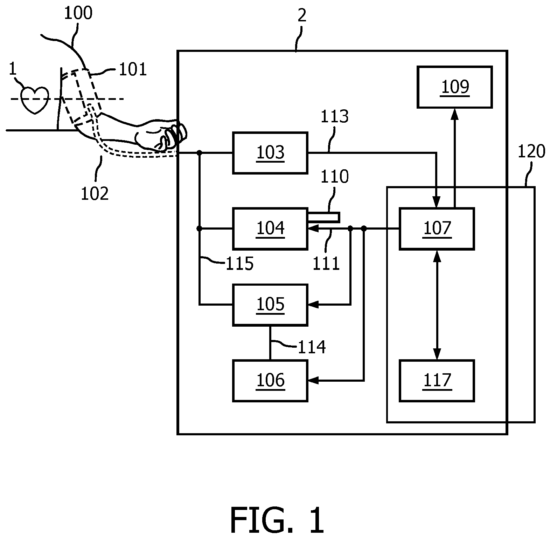

Referring to **FIG. 1****,** in one embodiment of the invention, a non-invasive blood pressure monitoring system includes an inflatable cuff 101 connected to the blood pressure monitoring system and attached to the measurement site; for example, an upper arm 100 of a patient 1. The system includes a display 109 for displaying measurement results, a main processor module 107, a memory unit 117 and an air system including: a source of pressured air 106, an optional inflate valve 105, deflate valve(s) 104, and pressure transducer(s) 103.

The blood pressure cuff 101 is connected by a hose 102 to the housing of system 2 and can be inflated and deflated for occluding the brachial artery of the patient 1 when in the fully inflated condition. As the blood pressure cuff 101 is deflated using deflate valves(s) 104 via an exhaust 110, the arterial occlusion is gradually relieved. The deflation of the blood pressure cuff 101 by the deflate valve(s) 104 is controlled by the central processor module 107 through a control line 111.

A pressure transducer 103 is coupled via the hose 102 to the blood pressure cuff 101 for sensing the pressure within the cuff 101. In accordance with conventional oscillometric techniques, the pressure transducer 103 is used to sense pressure oscillations in the cuff 101 that are generated by pressure changes in the artery under the cuff. The electrical oscillation signals from the pressure transducer 103 are obtained by the central processor module 107, using an analog to digital converter through a connection line 113.

The source of compressed air 106 comprises a pump or gas cylinder filled with compressed air. The compressed air is supplying the pressured air via duct 114 to the inflate valve(s) 105. The operation of the inflate valve(s) 105 or source of pressurized air 106 is controlled by the central processor module 107 through the control line 111. Thus, the inflation and deflation of the blood pressure cuff 101 is controlled by the central processor 107 through the deflate valve(s) 104 and the inflate valve(s) (105) or source of pressurized air 106, respectively.

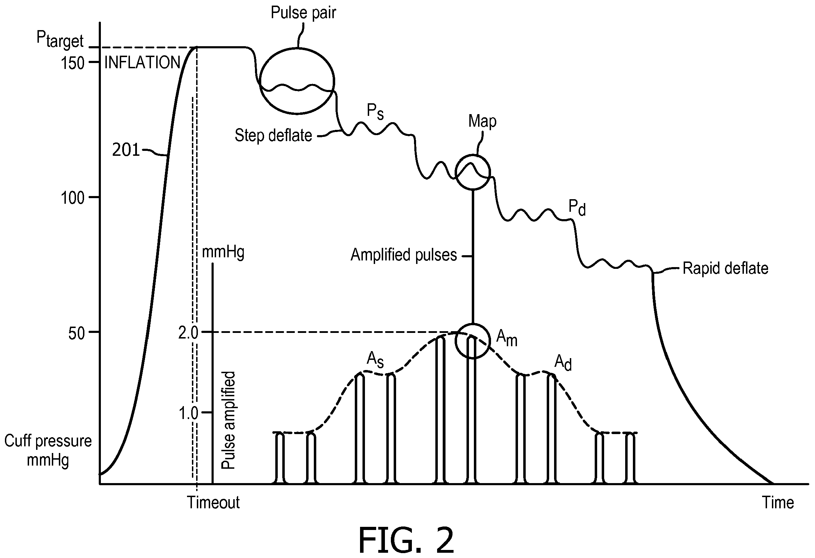

**FIG 2** illustrates the measurement cycle for oscillometric blood pressure. The oscillometric method measures blood pressure by monitoring the pulsatile changes in pressure that are caused by the flow of blood through an artery that is restricted by an occluding cuff. The cuff pressure for a measurement cycle, as measured by the transducer, is characterized by the wave 201. The cuff pressure rapidly increases to a maximum above the patient's systolic Pₛ pressure and is then deflated in a sequence of steps to a point below the diastolic pressure P_{d}. As the pressure in the cuff decreases, blood begins to flow through the artery. The sensitive transducer measures cuff pressure and small pressure oscillations within the cuff. A typical determination takes 10-12 deflation steps. Each step is made sufficiently long enough to include at least one heartbeat. As the pressure in the cuff decreases further, the pulses reach maximum amplitude Am. The pressure in the cuff that corresponds to the point of the maximum oscillation has been shown to correlate to a patient's mean arterial pressure (MAP). As the pressure in the cuff is decreased further, the pulses begin to decrease in amplitude (A_{d}). The rising and falling amplitude of the pressure pulses creates an envelope that is used to determine the patient's systolic (Ps), mean (Pm) and diastolic (Pd) pressures. The algorithm determines a patient's systolic and diastolic pressures by locating the points on the pulse pressure envelope that correspond to a predetermined percentage of the maximum amplitude (Aₘ). NIBP measurements can be taken manually, i.e. when each time only one measurement is taken, and automatically, when the measurement is repeated at specified intervals. If an automatic measurement series or programmable measurement series is running, the NIBP device according to various embodiments of the present invention measures the duration from the start of the inflation until a predefined cuff pressure level is reached (P_{target}). If this duration has increased significantly from the previous to the current inflation, the NIBP monitor indicates that the cuff has been disconnected from the device or is no longer applied to the patient's limb, aborts the current inflation, stops the automatic measurement series or programmable measurement sequence and issues a technical alarm; e.g. "Check Cuff".

Furthermore, the verification of whether or not an inflatable cuff is attached to the measurement site of a patient is performed during the inflation process. When performing the verification, the processor 107 checks whether the difference between the cuff inflation parameter value determined during the corresponding inflation operation and the cuff inflation parameter value determined during a preceding inflation operation meets a predetermined criterion. If the predetermined conditions are met, the measurements continue and, if not, the measurements terminate.

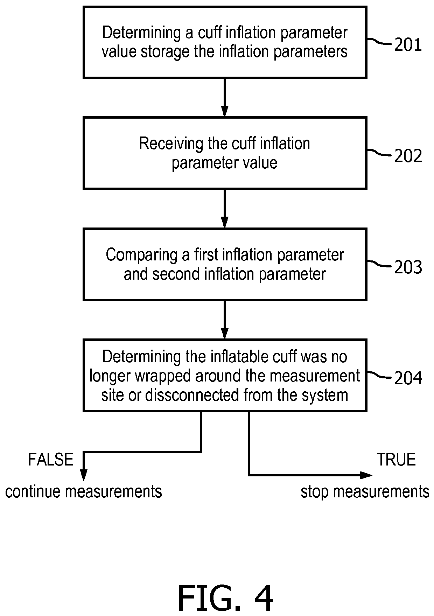

**FIG. 4** shows a diagram illustrating a method, performed by the blood pressure monitoring system 2. In the first step 201, the cuff inflation parameter values are determined in the processor 107 and stored in the memory unit 117. In the next step 202, the processor 107 reads the cuff inflation parameter values determined during two corresponding inflation operations. The processor 107 also compares the first inflation parameter with the second one in step 203. Based on the result of the comparison, the processor 107 decides in step 204 whether to continue or stop the measurements.

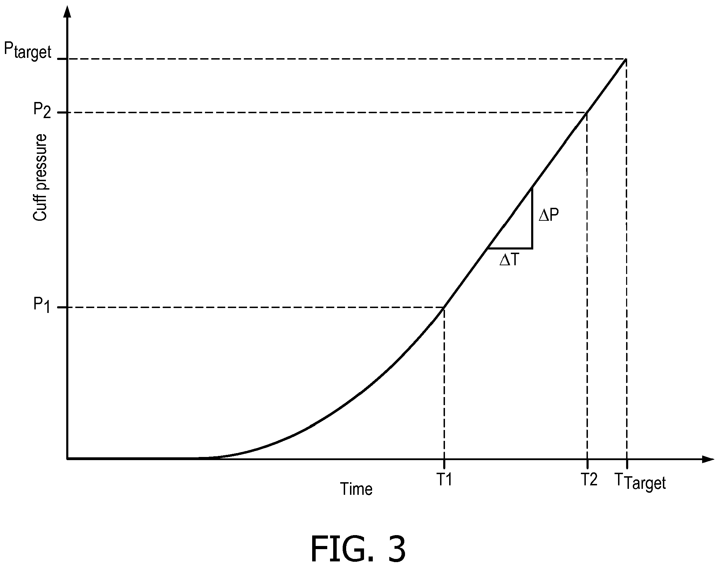

Referring again to **FIG. 2****,** cuff pressure dependency over time during a blood pressure measurement operation is shown. The graph illustrates the time available for the inflation to a target cuff pressure P_{target}, called timeout, which should be long enough to allow the inflation of the largest supported cuff loosely wrapped around the largest supported limb to the highest cuff pressure. The timeout is dependent on some variables, such as cuff size, the power of the source of pressured air, how tight the cuff is wrapped around a limb, the target cuff pressure, etc. Since it is not desirable to run a source of pressured air 106 without stopping, a timeout is set as a kind of safety mechanism that stops the source of pressured air 106 after the maximum expected time is over. Consequently, the timeout is not suitable to detect whether the cuff 101 is wrapped around a limb for smaller cuffs 101 because a smaller cuff 101 can be inflated to the target pressure even when it not wrapped around a limb. In the non-invasive blood pressure monitoring system 2 shown in **FIG. 1****,** a subsystem 120, comprising the processor 107 and the memory unit 117, measures the speed-dependent parameter when an automatic measurement series or programmable measurement series is running. As a speed-dependent parameter, a period of time required during the inflation operation that brings the pressure of the cuff 101 from the first level (P1) to the second level (P2) (see **FIG. 3****)** can be used. In a first embodiment, the first pressure level is slightly above the pressure of the cuff 101 when an inflation process starts (t=0), and the second pressure level is any pressure between the first pressure level and the target cuff pressure P_{target} at the end of the inflation operation. Preferably, the second pressure level is substantially smaller than the target cuff pressure P_{target} at the end of the inflation operation, such that an unnecessary inflation stops as soon as possible if the cuff is dislodged from the desired measurement site. This means that the second pressure level is selected such a patient feels no or only limited discomfort in his or her arm until the inflation process stops, if the cuff is accidentally or intentionally moved from the intended measurement site.In another embodiment, the inflation speed-dependent parameter is the first derivative dP/dt of the cuff pressure during inflation.

The processor 107 receives the inflation speed-dependent parameters and when a blood pressure monitoring system is in the automatic measurement series or programmable measurement series mode, the processor checks the difference between the cuff inflation parameter values determined during corresponding inflation operations in the measurement series. The processor 107 stops the inflation operation if the difference between the inflation speed-dependent parameter of the corresponding and the previous inflation operations exceeds a predetermined criterion.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein.

It should also be understood that, unless clearly indicated to the contrary, in any methods claimed herein that include more than one step or act, the order of the steps or acts of the method is not necessarily limited to the order in which the steps or acts of the method are recited.

## Claims

1. A detection device for use in a blood pressure measurement system, said device being arranged for coupling to an inflatable cuff attached to a measurement site of a body part of a patient and arranged to perform a series of blood pressure measurements, wherein, for each measurement of the series, an inflation operation is performed to inflate the cuff, the device comprising:
a determining unit arranged for determining an inflation speed-dependent parameter value during each inflation operation of the series,
a memory unit (117) arranged to store the determined inflation speed-dependent parameter value; and
a processor module arranged for receiving the determined inflation speed-dependent parameter values from the memory unit; and for checking whether a difference between the inflation speed-dependent parameter values determined during a first inflation operation and during a second inflation operation meets a predetermined criterion, wherein in the series of blood pressure measurements the second inflation operation is subsequent to the first inflation operation; and
wherein the processor module is further arranged to stop the inflation operation if the difference between the inflation speed-dependent parameter values exceeds the predetermined criterion.

2. The device of claim 1, wherein the second inflation operation immediately follows the first inflation operation in the series of blood pressure measurements.

3. The device of claim 1 or 2, wherein the inflation speed-dependent parameter value is defined as a period of time required during the inflation operation to bring the pressure of the cuff from a first pressure level to a second pressure level.

4. The device of claim 3, wherein the first pressure level is a pressure of the cuff at a start of the inflation operation.

5. The device of claim 3, wherein the second pressure level is a pressure level between the first pressure level and a target cuff pressure level at the end of the inflation operation.

6. The device of claim 5, wherein the second pressure level is substantially smaller than the target cuff pressure at the end of the inflation operation.

7. The device as claimed in any one of the preceding claims, wherein the memory unit is further arranged for storing reference speed-dependent parameter values for different cuff sizes, and wherein the device is arranged to compare the measured inflation speed-dependent parameter value with a selected one of the reference inflation-speed dependent parameter values stored in the memory.

8. The device of claims 1 and 2, wherein the inflation speed-dependent parameter value is the first derivative (dP/dt) of the cuff pressure during inflation.

9. A blood pressure measurement system comprising the device according to any one of claims 1 to 8, an inflatable cuff and a hose, wherein the device is coupled to the cuff via the hose.

10. A method of checking whether an inflatable cuff has been disconnected from or has been attached to a measurement site of a body part of a patient, the method being performed by a device for use in a blood pressure measurement system, coupled to the inflatable cuff attached to a measurement site of a body part of a patient, wherein the device performs a series of blood pressure measurements, wherein for each measurement of the series an inflation operation is performed to inflate the cuff, the method comprising the steps of:
determining an inflation speed-dependent parameter value during each inflation operation of the series,
storing the determined inflation speed-dependent parameter value in a memory unit;
receiving the determined inflation speed-dependent parameter values from the memory unit, and
determining a difference between the inflation speed-dependent parameter values determined during a first inflation operation and a second inflation operation, wherein in the series of blood pressure measurements the second inflation operation is subsequent to the first inflation operation; and wherein the method further comprises
stopping the inflation operation if the difference between the inflation speed-dependent parameter values exceeds a predetermined criterion.

## Patentansprüche

1. Detektionsvorrichtung zur Verwendung in einem Blutdruckmesssystem, wobei die Vorrichtung zum Koppeln mit einer aufblasbaren Manschette angeordnet ist, die an einer Messstelle eines Körperteils eines Patienten angebracht wird, und angeordnet ist, um eine Reihe von Blutdruckmessungen durchzuführen, wobei für jede Messung der Reihe ein Aufblasvorgang durchgeführt wird, um die Manschette aufzublasen, wobei die Vorrichtung umfasst:
eine Bestimmungseinheit, die zum Bestimmen eines von der Aufblasgeschwindigkeit abhängigen Parameterwertes während jedes Aufblasvorgangs der Reihe angeordnet ist,
eine Speichereinheit (117), die angeordnet ist, um den bestimmten, von der Aufblasgeschwindigkeit abhängigen Parameterwert zu speichern; und
ein Prozessormodul, das zum Empfangen der bestimmten, von der Aufblasgeschwindigkeit abhängigen Parameterwerte von der Speichereinheit angeordnet ist; und zum Überprüfen, ob eine Differenz zwischen den von der Aufblasgeschwindigkeit abhängigen Parameterwerten, die während eines ersten Aufblasvorgangs und während eines zweiten Aufblasvorgangs bestimmt wurden, ein vorbestimmtes Kriterium erfüllt, wobei in der Reihe von Blutdruckmessungen der zweite Aufblasvorgang auf den ersten Aufblasvorgang folgt; und
wobei das Prozessormodul weiter angeordnet ist, um den Aufblasvorgang zu stoppen, wenn die Differenz zwischen den von der Aufblasgeschwindigkeit abhängigen Parameterwerten das vorbestimmte Kriterium überschreitet.

2. Vorrichtung nach Anspruch 1, wobei der zweite Aufblasvorgang unmittelbar auf den ersten Aufblasvorgang in der Reihe von Blutdruckmessungen folgt.

3. Vorrichtung nach Anspruch 1 oder 2, wobei der von der Aufblasgeschwindigkeit abhängige Parameterwert als eine Zeitspanne definiert ist, die während des Aufblasvorgangs erforderlich ist, um den Druck der Manschette von einem ersten Druckniveau auf ein zweites Druckniveau zu bringen.

4. Vorrichtung nach Anspruch 3, wobei das erste Druckniveau ein Druck der Manschette zu Beginn des Aufblasvorgangs ist.

5. Vorrichtung nach Anspruch 3, wobei das zweite Druckniveau ein Druckniveau zwischen dem ersten Druckniveau und einem Zieldruckniveau der Manschette am Ende des Aufblasvorgangs ist.

6. Vorrichtung nach Anspruch 5, wobei das zweite Druckniveau im Wesentlichen kleiner ist als der Zieldruck der Manschette am Ende des Aufblasvorgangs.

7. Vorrichtung nach einem der vorstehenden Ansprüche, wobei die Speichereinheit weiter zum Speichern von geschwindigkeitsabhängigen Referenzparameterwerten für unterschiedliche Manschettengrößen angeordnet ist, und wobei die Vorrichtung angeordnet ist, um den gemessenen, von der Aufblasgeschwindigkeit abhängigen Parameterwert mit einem ausgewählten der im Speicher gespeicherten, von der Aufblasgeschwindigkeit abhängigen Referenzparameterwerte zu vergleichen.

8. Vorrichtung nach den Ansprüchen 1 und 2, wobei der von der Aufblasgeschwindigkeit abhängige Parameterwert die erste Ableitung (dP/dt) des Manschettendrucks während des Aufblasens ist.

9. Blutdruckmesssystem, das die Vorrichtung nach einem der Ansprüche 1 bis 8, eine aufblasbare Manschette und einen Schlauch umfasst, wobei die Vorrichtung über den Schlauch mit der Manschette gekoppelt ist.

10. Verfahren zum Überprüfen, ob eine aufblasbare Manschette von einer Messstelle eines Körperteils eines Patienten getrennt wurde oder an dieser angebracht wurde, wobei das Verfahren von einer Vorrichtung zur Verwendung in einem Blutdruckmesssystem durchgeführt wird, die mit der aufblasbaren Manschette gekoppelt ist, die an einer Messstelle eines Körperteils eines Patienten angebracht ist, wobei die Vorrichtung eine Reihe von Blutdruckmessungen durchführt, wobei für jede Messung der Reihe ein Aufblasvorgang durchgeführt wird, um die Manschette aufzublasen, wobei das Verfahren die folgenden Schritte umfasst zum:
Bestimmen eines von der Aufblasgeschwindigkeit abhängigen Parameterwertes während jedes Aufblasvorgangs der Reihe,
Speichern des bestimmten, von der Aufblasgeschwindigkeit abhängigen Parameterwertes in einer Speichereinheit;
Empfangen der bestimmten, von der Aufblasgeschwindigkeit abhängigen Parameterwerte von der Speichereinheit, und
Bestimmen einer Differenz zwischen den von der Aufblasgeschwindigkeit abhängigen Parameterwerten, die während eines ersten Aufblasvorgangs und eines zweiten Aufblasvorgangs bestimmt wurden, wobei in der Reihe von Blutdruckmessungen der zweite Aufblasvorgang auf den ersten Aufblasvorgang folgt; und wobei das Verfahren weiter umfasst
Stoppen des Aufblasvorgangs, wenn die Differenz zwischen den von der Aufblasgeschwindigkeit abhängigen Parameterwerten ein vorbestimmtes Kriterium überschreitet.

## Revendications

1. Dispositif de détection destiné à être utilisé dans un système de mesure de pression artérielle, ledit dispositif étant agencé pour être couplé à un brassard gonflable fixé à un site de mesure d'une partie du corps d'un patient et agencé pour effectuer une série de mesures de pression artérielle, dans lequel, pour chaque mesure de la série, une opération de gonflage est effectuée pour gonfler le brassard, le dispositif comprenant :
une unité de détermination agencée pour déterminer une valeur de paramètre dépendant de la vitesse de gonflage lors de chaque opération de gonflage de la série,
une unité mémoire (117) conçue pour stocker la valeur de paramètre déterminée en fonction de la vitesse de gonflage ; et
un module processeur conçu pour recevoir les valeurs de paramètres déterminées en fonction de la vitesse de gonflage à partir de l'unité mémoire ; et pour vérifier si une différence entre les valeurs de paramètres dépendant de la vitesse de gonflage déterminées pendant une première opération de gonflage et pendant une seconde opération de gonflage répond à un critère prédéterminé, dans lequel, dans la série de mesures de pression artérielle, la seconde opération de gonflage est postérieure à la première opération de gonflage ; et
dans lequel le module processeur est en outre agencé pour arrêter l'opération de gonflage si la différence entre les valeurs de paramètres dépendant de la vitesse de gonflage dépasse le critère prédéterminé.

2. Dispositif selon la revendication 1, dans lequel la seconde opération de gonflage suit immédiatement la première opération de gonflage dans la série de mesures de pression artérielle.

3. Dispositif selon la revendication 1 ou 2, dans lequel la valeur de paramètre dépendant de la vitesse de gonflage est définie comme une période de temps nécessaire pendant l'opération de gonflage pour amener la pression du brassard d'un premier niveau de pression à un second niveau de pression.

4. Dispositif selon la revendication 3, dans lequel le premier niveau de pression est une pression du brassard au début de l'opération de gonflage.

5. Dispositif selon la revendication 3, dans lequel le second niveau de pression est un niveau de pression compris entre le premier niveau de pression et un niveau de pression cible du brassard à la fin de l'opération de gonflage.

6. Dispositif selon la revendication 5, dans lequel le second niveau de pression est sensiblement inférieur à la pression cible du brassard à la fin de l'opération de gonflage.

7. Dispositif selon l'une quelconque des revendications précédentes, dans lequel l'unité mémoire est en outre agencée pour stocker des valeurs de paramètres de référence dépendant de la vitesse pour différentes tailles de brassard, et dans lequel le dispositif est agencé pour comparer la valeur mesurée de paramètre dépendant de la vitesse de gonflage avec l'une sélectionnée des valeurs de paramètres de référence dépendant de la vitesse de gonflage stockées dans la mémoire.

8. Dispositif selon les revendications 1 et 2, dans lequel la valeur de paramètre dépendant de la vitesse de gonflage est la première dérivée (dP/dt) de la pression du brassard pendant le gonflage.

9. Système de mesure de pression artérielle comprenant le dispositif selon l'une quelconque des revendications 1 à 8, un brassard gonflable et un tuyau, dans lequel le dispositif est couplé au brassard via le tuyau.

10. Procédé permettant de vérifier si un brassard gonflable a été détaché de ou a été fixé à un site de mesure d'une partie du corps d'un patient, le procédé étant exécuté par un dispositif destiné à être utilisé dans un système de mesure de pression artérielle, couplé au brassard gonflable fixé à un site de mesure d'une partie du corps d'un patient, dans lequel le dispositif effectue une série de mesures de pression artérielle, dans lequel pour chaque mesure de la série une opération de gonflage est effectuée pour gonfler le brassard, le procédé comprenant les étapes de :
déterminer une valeur de paramètre dépendant de la vitesse de gonflage lors de chaque opération de gonflage de la série,
stocker la valeur de paramètre déterminée dépendant de la vitesse de gonflage dans une unité mémoire ;
recevoir les valeurs de paramètres déterminées dépendant de la vitesse de gonflage à partir de l'unité mémoire, et
déterminer une différence entre les valeurs de paramètres dépendant de la vitesse de gonflage déterminées lors d'une première opération de gonflage et d'une seconde opération de gonflage, dans lequel dans la série de mesures de pression artérielle, la seconde opération de gonflage est postérieure à la première opération de gonflage ; et dans lequel le procédé comprend en outre
l'arrêt de l'opération de gonflage si la différence entre les valeurs de paramètres dépendant de la vitesse de gonflage dépasse un critère prédéterminé.
